# EUROPEAN PATENT APPLICATION

(11) **EP 1 612 553 A1**
(43) Date of publication of application: **04.01.2006**
(21) Application number: 05254018.4
(22) Date of filing: 28.06.2005
(51) Int. Cl.: G01N 33/487, B01L 11/00, C12Q 1/00, A61B 5/145

(54) **Analyte measuring apparatus which prevents the reuse of a test strip**

(30) Priority: 29.06.2004 US 882044
(71) Applicant: LifeScan, Inc., Milpitas, CA 95035 (US)
(72) Inventor: Allen, John J., Mendota Heights Minnesota 55118 (US)
(74) Representative: Mercer, Christopher Paul

(57) **Abstract**

The present invention may be used in test strips for measuring an analyte or indicator such as glucose in a physiological fluid such as blood, interstitial fluid, or urine. The present invention also relates to test strips incorporating an integrated lance such as a needle, blade, or other sharp or skin puncturing device. In particular, a fused link is incorporated into the test strip. The fused link may be destroyed once the test is completed, preventing reuse of the strip.

## Description

### CROSS-REFERENCE

This application is related to co-pending international application serial number PCT/GB01/05634, filed on December 19, 2001, entitled "Analyte Measurement" (published as WO 02/49507) which is hereby incorporated herein by reference.

### BACKGROUND OF THE INVENTION

The present invention relates, in general, to test strips for measuring an analyte or indicator, such as glucose, in a physiological fluid such as blood, interstitial fluid, or urine. More particularly, the present invention relates to test strips incorporating a system which prevents the re-use of such test strips.

The present invention may be used in test strips for measuring an analyte or indicator such as glucose in a physiological fluid such as blood, interstitial fluid, or urine. The present invention also relates to test strips incorporating an integrated lance such as a needle, blade, or other sharp or skin puncturing device. Certain types of medical devices such as, for example, glucose test strips were intended to be tested only once and then disposed. This requirement is often needed because the reagent chemistry in many test strips is not suitable for measuring glucose a second time. However, it is possible that some users will accidentally test a previously used test strip. This could potentially become a problem if the glucose meter attempts to make a glucose measurement and outputs a result. Therefore, it is desirable that a single use test strip and meter have a mechanism for preventing a previously tested test strip from being reused.

Recently, micro-needles (e.g. lances) and test strips (e.g., electrochemical-based and photometric-based biosensors) have been integrated into a single medical device. These integrated medical devices can be employed, along with an associated meter, to monitor various analytes, including glucose. Depending on the situation, biosensors can be designed to monitor analytes in an episodic single-use format, semi-continuous format, or continuous format. The integration of a micro-needle and biosensor simplifies a monitoring procedure by eliminating the need for a user to coordinate the extraction of a sample from a sample site with the subsequent transfer of that sample to a biosensor. This simplification, in combination with a small micro-needle and a small sample volume, also reduces pain.

For the case in which test strips are integrated with a lancing device, there is an added potential problem in that the re-use of test strips may result in cross-contamination. The lancing portion of the integrated device may have blood remaining on it which could infect a second user who might accidentally use the test strip. Therefore, it is desirable that the meter and test strip system have a mechanism which prevents a previously used test strip from launching the lance mechanism.

### SUMMARY OF THE INVENTION

In one embodiment of an analyte measurement system which prevents the reuse of a test strip according to the present invention, an analyte measuring system comprises a test strip for measuring an analyte electrochemically. In this embodiment of the invention, the test strip includes a frangible link disposed on the test strip. In a further embodiment, the frangible link comprises a conductive trace wherein the conductive trace is a material chosen from a group consisting of carbon, silver, platinum, palladium, gold, Ir, Pt, tungsten, copper, and aluminum. In a further embodiment of the present invention, the conductive trace has a positive temperature coefficient of resistance and includes a first electrical contact zone and second electrical contact zone, each adapted to receive a predetermined voltage from a meter.

In a further embodiment of the present invention, the conductive trace comprises a fuse zone located between the first and second electrical contact zone, wherein the fuse zone has a higher resistance than the first electrical contact zone and the second electrical contact zone. In a further embodiment the fuse zone melts when a predetermined voltage is applied between the first and second electrical contacts wherein the predetermined voltage ranges from about 1.5 volts to about 30 volts.

Further embodiments of the present invention may include: an analyte measuring system wherein the test strip includes an integrated lance; an analyte measuring system wherein the analyte is glucose; an analyte measuring system wherein the test strip includes a working electrode and a reference electrode; an analyte measuring system wherein a reagent layer is disposed on at least a portion of the working electrode; an analyte measuring system wherein the reagent layer comprises a redox mediator and a redox enzyme; and an analyte measuring system wherein the reagent layer comprises a silica filler.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 is a top exploded perspective view of a test strip embodiment having an integrated lance and a fuse;

Figure 2A is a partial plane view of a fuse which has a continuous conductive path;

Figure 2B is a partial plane view of a fuse which has a discontinuous conductive path;

Figure 3 is a bottom perspective view of a top layer of the test strip embodiment having an integrated lance;

Figure 4 is a flow chart illustrating the method of the present invention;

Figure 5 is a simplified schematic of a meter adapted for establishing electrical contact with a test strip of the present invention; and

Figure 6 is a simplified schematic of a meter interfaced with a test strip of the present invention.

### DETAILED DESCRIPTION OF ILLUSTRATIVE EMBODIMENTS OF THE INVENTION

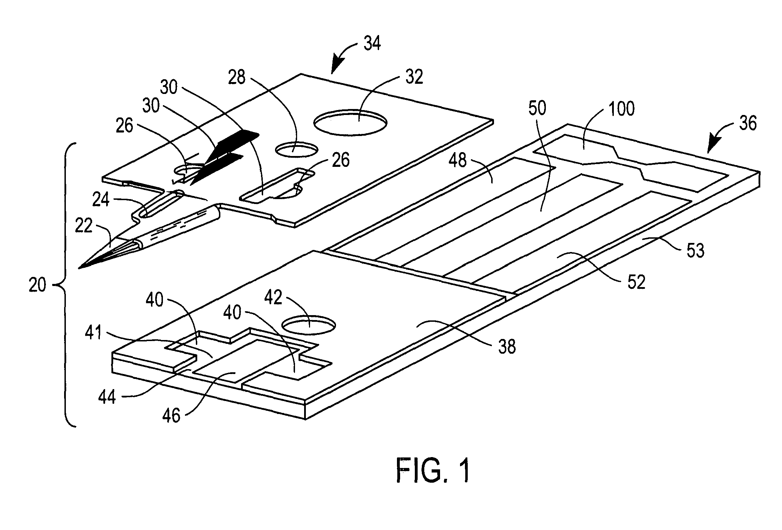

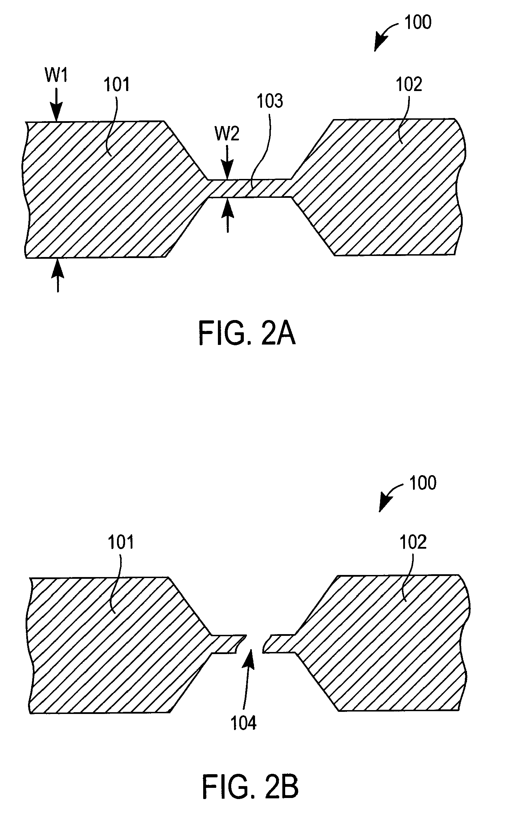

Figure 1 is a top perspective view of a test strip 20 according to the present invention. In this embodiment test strip 20 includes a first portion, in this case a top layer 34; a fixing mechanism, in this case an adhesive layer 38; and a second portion, in this case a bottom layer 36. In this example embodiment, bottom layer 36 includes a conductive layer which is deposed on a substrate 53. The conductive layer includes a first working electrode 48, a second working electrode 50, a reference electrode 52, and a frangible mechanism such as a fuse 100 here in the form of a frangible conductive pad. First working electrode 48, second working electrode 50, and reference electrode 52 may be in the form of a conductive pad. Top layer 34 includes the roof of sample receiving chamber 41. In an embodiment of the present invention, top layer 34 further includes an integrated lance 22, a stiffening rib 24, side embossment spacers 26, vents 30, a distal embossment spacer 28, and a registration hole 32 as shown in Figure 2. It should be noted that top layer 34 which incorporates integrated lance 22 may also known as a lancing first portion.

Test strip 20, which may be rectangular or another shape, is constructed by using a fixing mechanism such as adhesive layer 38 to attach top layer 34 to bottom layer 36. In an embodiment of the invention, test strip 20 may have an approximate width of 0.22 inches (i.e. 5.6 mm) and an approximate length of 0.55 inches (i.e. 14 mm). In the embodiment of Figure 1, the proximal end of test strip 20 includes fuse 100, while the distal end of test strip 20 includes integrated lance 22.

Test strip 20 further includes a sample receiving chamber 41 which is formed by the aggregate lamination of bottom layer 36, adhesive layer 38, and top layer 34 which represent the respective floor, wall, and roof of sample receiving chamber 41. Test strip 20 may be, for example, a glucose test strip which uses electrochemistry to measure the amount of glucose in a bodily fluid, such as, for example, blood or interstitial fluid. Alternatively or additionally, test strip 20 may be, for example, a coagulation sensor which measures a physical characteristic of a body fluid such as viscosity, capacitance, resistance, and the like.

The use of integrated lance 22 in test strip 20 makes testing simpler by eliminating the step of manually transferring sample into sample receiving chamber 41. Many previous sensor systems require a lancing step using a dedicated lancing device followed by the manual manipulation of the test strip so that it can be dosed with sample. The use of integrated lance 22 allows fluid to seamlessly flow from the wound to sample receiving chamber 41 without removing integrated lance 22.

In an embodiment of the present invention, fuse 100 is deposed on substrate 53 by a process such as, for example, screen printing, sputtering, evaporation, electroless plating, ink jetting, sublimation, chemical vapor deposition, and the like. The geometry of fuse 100 may be formed by using a screen which selectively allows conductive material to pass through in a defined pattern such as the one shown in Figure 2. Suitable materials which may be used for fuse 100 are carbon, silver, platinum, palladium, gold, Ir, Pt, tungsten, copper, aluminum, and the like. In an embodiment of this invention, fuse 100 may be deposed during the same print cycle that deposes first working electrode 48, second working electrode 50, and reference electrode 52, and thus, shows that the process of making fuse 100 may be simple and inexpensive to implement.

As shown in Figure 1, fuse 100 is located on the proximal end of test strip 20 which is the end farthest away from integrated lance 22. Fuse 100 includes a first electrical contact zone 101, a second electrical contact zone 102, and a fuse zone 103. First electrical contact zone 101 and second electrical contact zone both have a width W1 and are positioned such that they can electrically interface with a meter which can apply a voltage therebetween. In an embodiment of this invention, fuse zone 103 may have a width W2 which is less than W1. In addition, fuse zone 103 is positioned in between first electrical contact zone 101 and second electrical contact zone. Fuse 100 may have a generally rectangular shape with a narrower or waisted width W2 which corresponds to fuse zone 103. Fuse zone 103 is designed to have a higher resistance than first electrical contact zone 101 and second electrical contact zone 102 so that fuse zone 103 will blow or ablate when a certain voltage is applied across first electrical contact zone 101 and second electrical contact zone 102. In an embodiment of the present invention, fuse zone 103 may have a resistance ranging from about 0.5 ohms to about 1000 ohms. Because fuse zone 103 has a higher resistance than first electrical contact zone 101 and second electrical contact zone 102, when an appropriate voltage is applied, fuse zone 103 will heat up and eventually melt, forming an open circuit.

As part of bottom layer 36, first working electrode 48, second working electrode pad 50, and reference electrode 52 are deposed on substrate 53. Similar to fuse 100, first working electrode 48, second working electrode 50, and reference electrode 52 may be deposited using one of the previously mentioned techniques described for fuse 100 and indeed may be manufactured or deposited at the same time. The geometry of first working electrode 48, second working electrode 50, and reference electrode 52 may be formed by using a screen which selectively allows conductive material to pass through in a defined pattern. Suitable materials which may be used for first working electrode 48, second working electrode 50, and reference electrode 52 are Au, Pd, Ir, Pt, Rh, silver, silver chloride, stainless steel, doped tin oxide, carbon, and the like. Possible embodiments of the electrode geometry suitable for use with the subject invention include those described in U.S. Patent Nos. 6,716,577; 6,620,310; 6,558,528; 6,475,372; 6,193,873; 5,708,247; 5,951,836; 6,241,862; 6,284,125; and 6,444,115, and International Patent Application Publications WO/0167099; WO/0173124; WO/0173109; and WO/0206806, the disclosures of which are herein incorporated by reference.

As part of bottom layer 36, substrate 53 may be an electrically insulating material such as plastic, glass, ceramic, and the like. In a preferred embodiment of this invention, substrate 53 may be a plastic such as, for example, nylon, polyester, polycarbonate, polyimide, polyvinylchloride, polyethylene, polypropylene, and PETG. In an embodiment of the invention, the material used for substrate 53 may be a polyester material (trade name Melinex ® ST328) which is manufactured by DuPont Teijin Films.

As part of the bottom layer 36, insulation layer 44 may be printed or disposed over a portion of the conductive layer in order to define the electrode area which is wetted by a liquid sample. In an embodiment of this invention insulation layer 44 may be printed by using one of the aforementioned techniques described for fuse 100. In a preferred embodiment of this invention, insulation layer 44 may be printed by using screen printing techniques in either a flat bed process or in a continuous web process. A suitable material which may be used for insulation layer 44 is Ercon E6110-116 Jet Black Insulayer Ink which may be purchased from Ercon, Inc. It should be appreciated that to one skilled in the art that several different types of insulating material could be suitable for use in the described invention. In an embodiment of this invention, insulation layer 44 may have a height between 1 and 100 microns, more favorably between 5 and 25 microns, and yet even more favorably at about 5 microns.

As part of the bottom layer 36, reagent layer 46 may be printed by using one of the aforementioned techniques described for fuse 100. In a preferred embodiment of this invention, reagent layer 46 may be printed by using screen printing techniques. A non-limiting example of a suitable reagent or enzyme ink for use in he present invention can be found in issued US patents 5,708,247 and 6,046,051; published international applications WO01/67099 and WO01/73124. In an embodiment of this invention where test strip 20 is a glucose sensor, reagent layer 46 may comprise a redox enzyme and a redox mediator. Examples of redox enzymes may include glucose oxidase, glucose dehydrogenase using either a methoxatin co-factor, or a nicotinamide adenine dinucleotide co-factor. Examples of redox mediators may include ferricyanide, phenazine ethosulphate, phenazine methosulfate, pheylenediamine, 1-methoxy-phenazine methosulfate, 2,6-dimethyl-1, 4-benzoquinone, 2,5-dichloro-1,4-benzoquinone, phenathiazine derivatives, phenoxazine derivatives, metalloporphyrin derivatives, phthalocyanine derivatives, viologen derivatives, ferrocene derivatives, osmium bipyridyl complexes, ruthenium complexes and the like. It should be appreciated that one skilled in the art that variations of the previously described enzyme ink could be suitable for use in the described invention. In an embodiment of this invention, reagent layer 46 may have a height between 1 to 100 microns, and more favorably between 5 to 25 microns.

In an embodiment of the present invention, adhesive layer 38 includes at least portion of the walls of a sample receiving chamber 41. Adhesive layer 38 may be printed or disposed on top of a portion of insulation layer 44 and/or a portion of reagent layer 46 to at least partially form a sample receiving chamber 41 within test strip 20. Examples of methods to print adhesive layer 38 may be screen printing, gravure, and slot coating. In other embodiments, adhesive layer 38 may be a double sided pressure sensitive adhesive, a UV cured adhesive, heat activated adhesive, or a thermosetting plastic. As a non-limiting example, adhesive layer 38 may be formed by screen printing a pressure sensitive adhesive such as, for example, a water based acrylic copolymer pressure sensitive adhesive which is commercially available from Tape Specialties LTD in Tring, Herts, United Kingdom as part #A6435.

In an embodiment of this invention, the height or adhesive layer 38 may be between 4 and 140 microns. The minimal value for the adhesive height is bounded by the height of reagent layer 46 because it would be undesirable for top layer 34 to physically contact reagent layer 46 and result in possible damage to reagent layer 46. The maximum value of the adhesive height is bounded by the desire to reduce the overall sample volume of test strip 20. Other factors which may influence the selected adhesive height may be the desire to maintain conditions for semi-infinite diffusion in regards to the mediator oxidation (i.e. concentration of redox mediator which is sufficiently far from the electrodes are unperturbed by electrochemical reactions).

In an embodiment of this invention, adhesive layer 38 further includes a side clearance area 40 and a distal clearance area 42. The clearance areas within the adhesive may be used to provide an area in which side embossment spacer 26 can interface with insulation layer 44 in such a manner that top layer 34 forms the roof of sample receiving chamber 41. Adhesive layer 38 should have at least about a slightly greater height than side embossment spacers 26 and distal embossment spacer 28 so that the embossment spacers provide a mechanical stop to limit the compression of the adhesive height between the top layer 34 and bottom layer 36. Therefore, the use of embossment spacers or other mechanical protrusions help control the sample chamber height when using either heat activated adhesive or thermosetting plastic.

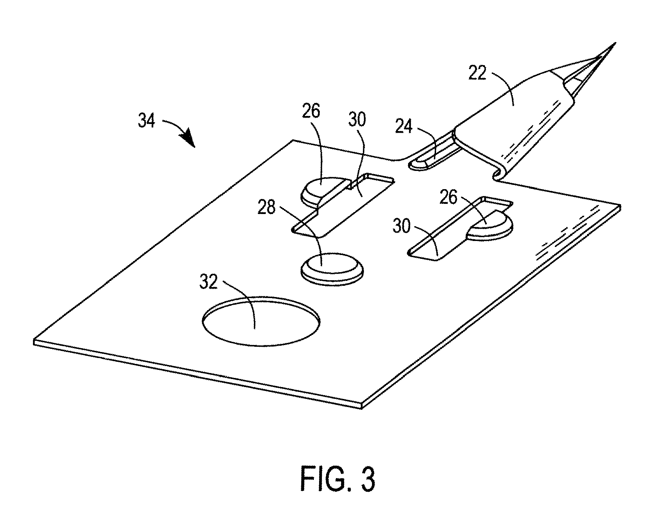

Figure 3 is a bottom perspective view of top layer 34 which illustrates the morphology of integrated lance 22, stiffening rib 24, side embossment spacer 26, and distal embossment spacer 28 from the bottom perspective view. Top layer 34 may be, for example, a sheet of conductive material such as gold, platinum, stainless steel, silver, and palladium, or other suitable metal which has the appropriate ductility to allow embossment. For the case using stainless steel, the metal may be plated with gold, platinum, stainless steel, silver, and palladium to reduce the costs of materials. The geometry of top layer 34, side embossment spacer 26, and distal embossment spacer 28 may be formed by, for example, a stamping process which may be performed by Meier Tool and Engineering (Anoka, Minnesota). The height of side embossment spacers 26 and distal embossment spacer 28 may range from about 4 to 130 microns, more preferably between about 50 to 110 microns, and yet more preferably between about 80 to 105 microns. Vent 30 may be formed by, for example, punching through top layer 34. In an embodiment of this invention vent 30 is adjacent to side embossment spacer 26. Vent 30 may be used to partially define a portion of the wall of sample receiving chamber 41 and to facilitate the transport of bodily fluid up integrated lance 22 and into sample receiving chamber 41. Registration hole 32 may be formed during the stamping process of making top layer 34.

As an embodiment of the present invention, integrated lance 22 may be manufactured as an integral part of top layer 34. Integrated lance 22 may be formed in a stamping process where it has a "V" shaped open channel geometry. More details concerning the design of integrated lance 22 may be found in US provisional application serial number 60/458,242 and 60/459,465 which are incorporated by reference herein. For certain embodiments of the invention, top layer 34 may be coated with a surfactant coating or undergo a hydrophilic surface treatment to in increase the capillary force of test strip 20. Non-limiting examples of surfactant coatings are Tween-80, JBR-515, Niaproof, and Tergitol. Integrated lance 22 may further include stiffening rib 24 as shown in Figure 1 and 3 which strengthens the structural integrity of integrated lance 22 and to assist with fluidic flow along integrated lance 22 to sample receiving chamber 41.

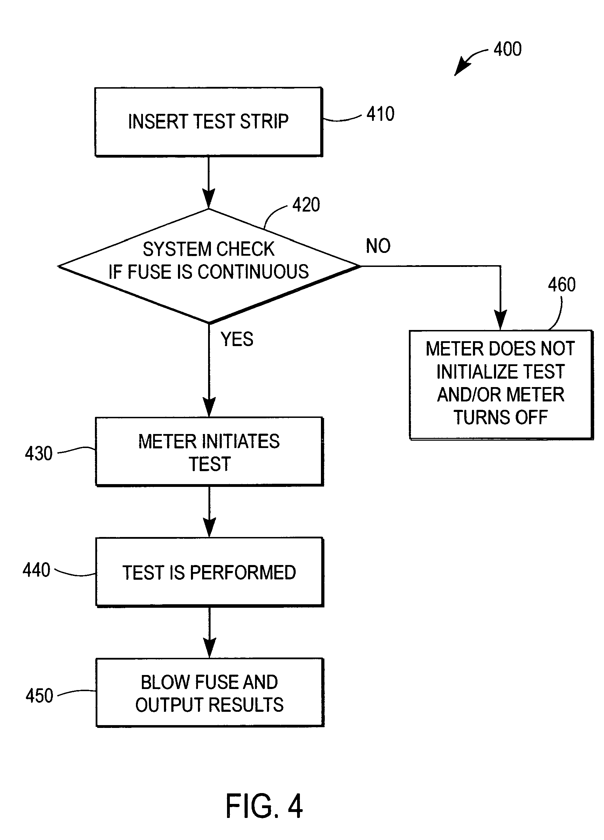

Figure 4 shows a flow chart 400 which describes a method of preventing the re-use of a test strip according to one embodiment of the present invention. In step 410, a meter interfaces with test strip 20 such that the meter establishes electrical contact with first working electrode 48, second working electrode 50, reference electrode 52, first electrical contact zone 101, and second electrical contact zone 102. Next, the meter performs a system check which includes probing the continuity of fuse 100 across first electrical contact 101 and second electrical contact 102 as illustrated in step 420. In step 430, if the meter determines that fuse 100 is continuous, then meter will turn on and/or initiate a test prompting the user to launch a lancing mechanism. For the case in which the fuse 100 is continuous, the meter will perform the test analyzing a physiological sample for step 440. Next, the meter will output a result of the analysis and then blow fuse 100. Figure 2B shows a partial plane view of a blown fuse which has a discontinuous zone 104. In alternative embodiments to the present invention, fuse 100 can be blown at any time after step 430 because this ensures that test strip 20 will not be reused after previous exposure to a physiological sample. In an embodiment of this invention, the meter can apply a constant voltage across first electrical contact zone 101 and second electrical contact zone 102 which may range from about 1.5 volts to about 30 volts. In another embodiment of this invention, the meter can apply a variable voltage for the purpose of applying a constant current across first electrical contact zone 101 and second electrical contact zone 102 which may range from about 20 microamps to about 1500 microamps. In summary, this method of the present invention provides a robust strategy for ensuring that a user can only use a test strip once.

In addition, this method of the present invention can determine if a test strip has been previously used and prevent the user from testing a used test strip. If the meter determines that fuse 100 is discontinuous, then the meter will turn off and/or output an error message indicative of defective/used test strip as shown in step 460.

The purpose of fuse 100 is to reduce and effectively prevent the possibility that test strip 20 is reused. An embodiment of this invention includes top layer 34 having an integrated lance 22. Therefore, the reuse of test strip 20 can result in cross-contamination of physiological fluid or infection to the user. Therefore, it is desirable to have fuse 100 which can allow a meter to determine if test strip 20 has already been tested. The meter is designed to break fuse 100, or in some cases blow a fuse, after test strip 20 has been tested. If the meter determines that test strip 20 has been already tested (e.g. by testing that the fuse 100 is broken or the fuse is blown), the meter will either output an error message and/or prevent initiation of the test. However, if the meter determines that test strip 20 has not been tested, the meter will initiate the test by either launching integrated lance 22 towards the skin or prompting the user to do so by actuating a switch.

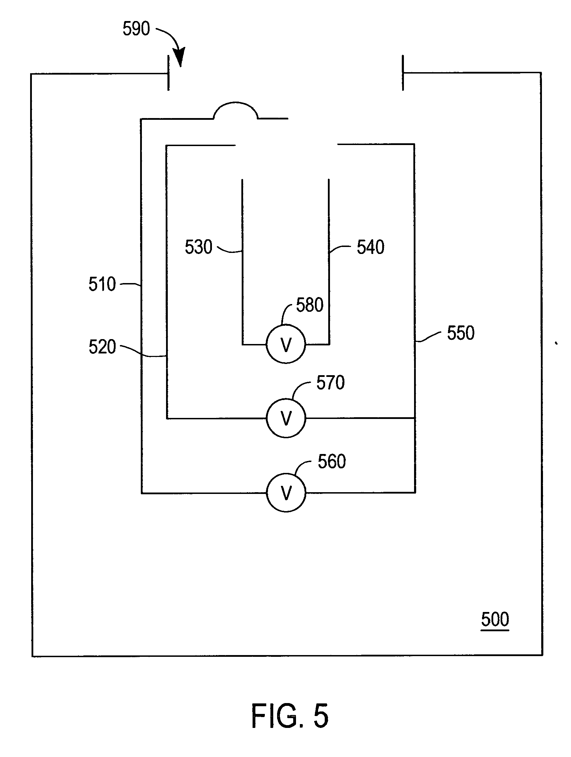

Figure 5 is a simplified schematic of a meter 500 adapted for establishing electrical contact with a test strip 20 of the present invention. Meter 500 includes a strip insertion port 590, a means for measuring glucose using either one or two working electrodes, a means for determining whether test strip 20 has been previously tested with a physiological fluid, and a means for blowing fuse 100.

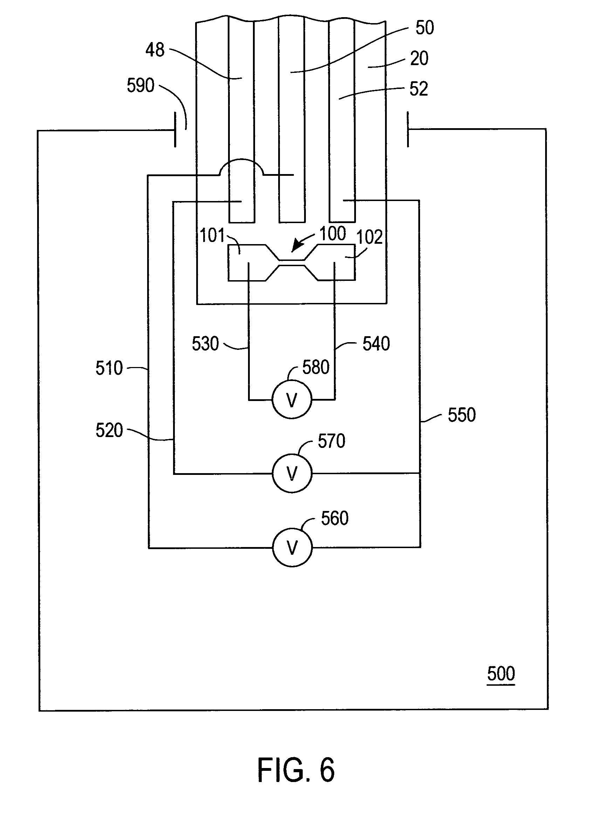

Strip insertion port 590 includes an opening or orifice within meter 500 that allows a portion of test strip 20 to be inserted into meter 500. More specifically, the proximal end of test strip 20 may be inserted into meter 500 such that electrical contact can be established with first working electrode 48, second electrode 50, reference electrode 52, and fuse 100. Figure 6 shows an example of meter 500 forming electrical contact with the proximal end of test strip 20.

The means for measuring glucose includes first working electrode contact 510, second working electrode contact 520, reference electrode contact 550, first test voltage source 560, and second test voltage source 570. Meter 500 is designed such that first working electrode contact 510, second working electrode contact 520, and reference electrode contact 550 establish electrical contact with first working electrode 48, second working electrode 50, and reference electrode 52, respectively, as shown in Figure 6. When performing a glucose measurement, first test voltage source 560 may apply a first voltage E1 between first working electrode 48 and reference electrode 52. In a similar manner, second test voltage source 570 may apply a second voltage E2 between second working electrode 50 and reference electrode 52. In an embodiment of this invention, E1 and E2 may range from about -100 millivolts to about 700 millivolts, and may more preferably range about 0 millivolts to about 400 millivolts. A physiological sample is applied such that first working electrode 48, second working electrode 50, and reference electrode 52 are covered with sample. In turn, this causes reagent layer 46 to become hydrated which generates ferrocyanide in an amount proportional to the glucose present in the sample. In an embodiment of this invention, meter 500 further includes the ability to measure current which allows an oxidation current for both first working electrode 48 and second working electrode 50 to be measured after about 5 seconds from the sample application. The measured currents may then be correlated to a glucose concentration value and which is displayed on a LCD screen of meter 500.

The means for determining whether test strip 20 has been previously tested with a physiological fluid includes a first continuity contact 530, a second continuity contact 540, and a continuity voltage source 580. Meter 500 is designed such that first continuity contact 530 and second continuity contact 540 establish electrical contact with first electrical contact zone 101 and second electrical contact zone 102, respectively, as shown in Figure 6. When inserting test strip 20 into meter 500, continuity voltage source 580 may apply a constant voltage E3 between first electrical contact zone 101 and second electrical contact zone 102. Next meter 500 interrogates test strip 20 for an electrical continuity between first electrical contact zone and second electrical contact zone which may determined by a measured current value (as opposed to a near zero current value). If fuse 100 is determined to be continuous, then the glucose measurement is allowed to initiate. If fuse 100 is determined to not be continuous, then the glucose measurement does not initialize and/or meter 500 turns off.

In an alternative embodiment to the present invention, continuity voltage source may apply a variable voltage such that a constant current is applied between first electrical contact zone 101 and second electrical contact zone 102. Next meter 500 interrogates test strip 20 for an electrical continuity between first electrical contact zone and second electrical contact zone which may determined by a measured non-infinite voltage value (as opposed to an infinite voltage value).

The means for blowing fuse 100 includes a voltage source or current source which may be applied across first continuity contact and second continuity contact. Because meter 500 is designed such that first continuity contact 530 and second continuity contact 540 establish electrical contact with first electrical contact zone 101 and second electrical contact zone 102, a sufficiently strong voltage or current

It is an advantage of this invention in that it is more reliable than existing techniques because it identifies a used test strip as soon as the test strip is inserted into the meter. This early detection capability is especially useful for test strips having an integrated lance 22 because reuse can be a source of contamination and infection.

It is an another advantage of this invention in that a used test strip can be identified by the meter even when the liquid sample applied to the test strip has dried. Impedance techniques for identifying a used test strip require liquid to be within the test strip.

It is another advantage of this invention in that a fuse can be added to the test strip at a low cost. It is a simple manufacturing step to print an additional electrode onto the test strip.

It is another advantage of this invention in that the circuitry required determining the continuity of a fuse is very simple and low cost.

While preferred embodiments of the present invention have been shown and described herein, it will be obvious to those skilled in the art that such embodiments are provided by way of example only. Numerous variations, changes, and substitutions will now occur to hose skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. An analyte measuring system comprising:
a test strip having a means for measuring an analyte electrochemically; and
a frangible link disposed on said test strip.

2. The analyte measuring system of Claim 1, wherein said frangible link comprises a conductive trace.

3. The analyte measuring system of Claim 2, wherein said conductive trace is a material chosen from a group consisting of carbon, silver, platinum, palladium, gold, Ir, Pt, tungsten, copper, and aluminum.

4. The analyte measuring system of Claim 2, wherein said conductive trace has a positive temperature coefficient of resistance.

5. The analyte measuring system of Claim 2, wherein said conductive trace comprises:
a first electrical contact zone and second electrical contact zone each adapted to receive a predetermined voltage from a meter; and
a fuse zone located in between said first electrical contact zone and said second electrical contact zone wherein said fuse zone has a higher resistance than said first electrical contact zone and said second electrical contact zone

6. The analyte measuring system of Claim 5, wherein said fuse zone becomes an open circuit when a predetermined voltage is applied between said first electrical contact and said second electrical contact.

7. The analyte measuring system of Claim 5, wherein said predetermined voltage ranges from about 1.5 volts to about 30 volts.

8. The analyte measuring system of Claim 1, wherein said test strip further comprises an integrated lance.

9. The analyte measuring system of Claim 1, wherein said analyte is glucose

10. The analyte measuring system of Claim 1, wherein said test strip further comprises a working electrode and a reference electrode

11. The analyte measuring system of Claim 10, wherein a reagent layer is disposed on at least a portion of said working electrode.

12. The analyte measuring system of Claim 11, wherein said reagent layer comprises a redox mediator and a redox enzyme.

13. The analyte measuring system of Claim 11, wherein said reagent layer comprises a silica filler.

14. A test strip for use in an analyte measurement system, said test strip comprising:
a plurality of electrical contacts;
a sample chamber adapted to receive a sample of a bodily fluid, wherein said sample chamber is connected to a first pair of said electrical contacts; and
a frangible link connected to a second pair of said electrical contacts.

15. A test strip according to Claim 14, wherein said frangible link comprises a fuse.

16. A test strip according to Claim 14, wherein said frangible link comprises a conductive trace having an impedance greater than the impedance of said electrical contacts.

17. A test strip according to Claim 14, wherein said frangible link is adapted to open when a predetermined voltage is applied to said second pair of electrical contacts.
